# EUROPEAN PATENT SPECIFICATION

(11) **EP 2 956 110 B1**
(45) Date of publication and mention of the grant of the patent: **12.08.2020**
(21) Application number: 14705960.4
(22) Date of filing: 12.02.2014
(51) Int. Cl.: A61K 6/00

(54) **RESORBABLE AND CURABLE COMPOSITIONS FOR USE IN DENTISTRY**
RESORBIERBARE UND HÄRTBARE ZUSAMMENSETZUNGEN ZUR VERWENDUNG IN DER ZAHNMEDIZIN
COMPOSITIONS RÉSORBABLES ET DURCISSABLES À UTILISER EN DENTISTERIE

(30) Priority: 14.02.2013 US 201361764539 P
(43) Date of publication of application: 23.12.2015
(73) Proprietor: DENTSPLY SIRONA Inc., York, PA 17401-2991 (US)
(72) Inventor: ANGELETAKIS, Christos, Orange, CA 92865 (US)
(74) Representative: Hartz, Nikolai
(86) International application number: PCT/US2014/015902
(87) International publication number: WO 2014/126945

(56) References cited:
- WO-A2-2005/009225
- US-A- 4 472 840
- US-A- 5 002 769
- US-A1- 2002 061 329
- DATABASE WPI Week 198650 Thomson Scientific, London, GB; AN 1986-329424 XP002723901, & JP S61 246107 A (SANKIN KOGYO KK) 1 November 1986 (1986-11-01)
- Joseph Muhammad ET AL: "The Use of a Bioadhesive (BioGlue ) Secured Conchal Graft and Mandibular Distraction Osteogenesis to Correct Pediatric Facial Asymmetry as Result of Unilateral Temporomandibular Joint Ankylosis", Craniomaxillofacial Trauma and Reconstruction, vol. 06, no. 01, 18 January 2013 (2013-01-18), pages 049-056, XP055490679, ISSN: 1943-3875, DOI: 10.1055/s-0032-1332208

## Description

### TECHNICAL FIELD

Provided herein is a two-part curable protein/aldehyde composition, such as bovine serum albumin/glutaraldehyde forming a protein hydrogel, used to form a biodegradable cavity liner_{;} Further disclosed heirein is a resorbing curable wound dressing or a biodegradable adhesive in dental restorative and endodontic procedures.

### BACKGROUND

JP S61246107 discloses material for restoration of hard tissue of the human body.

US 4472840 discloses reconstituted collagen to induce osseous formation by implanting bone graft material.

US 5002769 discloses biodegradable compositions for the sustained release of chlorhexidine.

US 2002/061329 discloses a polymer-based wound dressing and drug delivery system.

The dental pulp is the part in the interior of a tooth made up of living connective tissue and cells, ground substance, and neural and vascular supplies. The pulp, in conjunction with the dentin that surrounds it, is referred to as the pulp-dentin complex. Dentin is a porous, fluid-filled mineralized tissue that provides critical mechanical support to overlying enamel and forms the bulk of the mineralized portion ofi the tooth.

Most cavities discovered during a dental examination will need to be treated. In general, if a cavity has broken through the enamel and is into the underlying dentin, or is able to be probed with an explorer, it has undergone cavitation, and requires treatment. During excavation of deep cavities or placement of crowns, it is customary to use a cap or liner over the exposed pulp or dentin before applying the final filling material. Dental liners provide a barrier against acidic monomers and etchants used in restorative procedures, may act as a barrier against ingress of decay-causing microorganisms, may stimulate formation of reparative dentin, and/or may provide thermal insulation. Direct pulp caps are more particularly used when both pink dentin and the pulp are exposed. However there is always the risk of causing inflammation or necrosis of the pulp, in which case endodontic procedures need to be performed and the pulp is removed.

The same dental material used for liners is also conventionally used for pulp capping to protect the pulp after excavating deep caries, which result in penetration near or into the pulp chamber. The pulp and the adjacent tissue, the pink dentin, are the living portion of a tooth, and accordingly, are highly prone to irritation and inflammation caused by many different components of dental materials, such as acidic monomers used as adhesives and other resinous components, and to destruction by pathogenic contamination. The failure to properly seal the pulp chamber and pink dentin can lead to bacterial infection of the pulp. Such infections are very difficult for the body to fight and may often lead to serious infections of the teeth and surrounding bone. Inflammation and infection of the pulp can lead to pulp death.

During restorative procedures, when the remaining dentin thickness (RDT), defined as the distance between the cavity floor and pulp tissue, is less than 0.5 mm, it is well known that ingredients of dental materials used in dental restorative procedures can penetrate into the pulp, inhibit cell metabolism and cause inflammation. (C. A. de Souza Costa et al., Dental Materials 18, (2002) 543-551, IADR poster No. 2416, IADR Gen. Meeting Seattle WA 2013 ). Acidic acrylic monomers and solvents, such as ethanol, that are commonly used in restorative dentistry increase the permeability of the dentin and also can contribute to the inflammation. The inflammation caused by the harsh chemicals can lead to pulp necrosis unless controlled. When there is direct pulp exposure, pulp withdrawal and necrosis is expected.

Currently available cavity liners such as DYCAL® (Dentsply) or LIFE® (Kerr) are alkaline and calcium releasing and may decrease the long-term inflammatory response possibly by stimulating the formation of reparative dentin. However the pulp withdraws during the process, with the general outcome of making the tooth more brittle and more subject to fracture. Portland cement or mineral trioxide aggregate (MTA) is an alternative pulp capping material that is more biocompatible, but it has a poor delivery system and a long setting time of approx. 4 hours. There is thus a need for a resorbable curable material that can be used as a cavity lining material in restorative dentistry for assisting the regeneration of the dental pulp by acting as a scaffold and that reliably seals the pulp cavity during a dental restorative procedure.

There can be other uses for a resorbing curable material in restorative dentistry. For example, there is a need in dentistry for a wound dressing that degrades over time. Post-surgical sites intraorally are subject to food bolus trauma as well as tooth brushing that can irritate the site and delay healing. A wound dressing that can remain in place after surgery to protect the site is important - if the dressing is additionally resorbing, then an additional benefit is obtained.

Another application for a resorbing curable material is for care of furcation involvement during endodontic surgery. The area at the root furcation can often have iatrogenic involvement extending through the tooth structure and into the underlying bone. Placing a resorbable material that is compatible with the bone is important dming the surgical treatment to seal off the area and promote healing. A related application would be anytime the root structure needs to receive palliative treatment - i.e. fracture, or again iatrogenic puncturing of the root during root canal treatment.

### SUMMARY

The present invention relates to the subject matter of claims 1 to 8.

The present disclosure provides a resorbing curable composition for use in an intraoral treatment or procedure, The resorbing curable cavity lining material is - protects the pulp during and after restorative procedures, such as from the harsh chemicals used in acid etching and leaching of dental monomers that lead to inflammation of the pulp and from bacteria. After the restoration is placed, this lining material slowly degrades while acting as a scaffold for new cell growth in the remaining space. In accordance with the present invention the resorbing material, such as a degradable cavity liner, is the product of a two-part curable protein/aldehyde composition.

According to the present invention, the composition is used in a method for the protection and regeneration of dental pulp in a dental restorative procedure, the method comprising preparing a tooth area to provide a cavity floor within the tooth, wherein a remaining dentin thickness between the cavity floor and the dental pulp is less than about 0.5 mm, and applying to the cavity floor a solution of a proteinaceous material and an aldehyde and allowing a polymerization reaction to occur to thereby fonn a protein hydro gel that can perform as a biodegradable cavity liner comprising the proteinaceous material in cross-linked form of gel consistency,

The method further comprises etching the remaining tooth area with an etchant, such as an aqueous phosphoric acid or polyacrylic acid solution, after applying the liner solution in the deepest part of the cavity and allowing it to cure, whereby the biodegradable cavity liner prevents the etchant from contacting the dental pulp, and applying a restorative composition to the tooth area after etching to restore the tooth. The biodegradable cavity liner will also prevent residual dental monomers in the restorative from leaching into the pulp casing inflammation.

In addition to the above, this resorbing curable material can have other applications in dentistry such as use as a post oral surgery dressing and adhesive and as a biodegradable adhesive for repair of tooth fractures. Thus, Also disclosed is a method for treating an intraoral surface, comprising applying to the intraoral surface a solution of a proteinaceous matelial and an aldehyde and allowing a polymerization reaction to occur to thereby form a biodegradable material comprising the proteinaceous material in crosslinked fonn.

### DETAILED DESCRIPTION

Provided herein is a resorbing curable material, such as a cavity lining material for use in restorative dentistry for reliably sealing the pulp cavity to prevent ingress of components of restorative materials and bacteria and assisting the regeneration of the dental pulp. To that end, a twopart curable protein/aldehyde composition, for example bovine serum albumin (hereinafter "BSA")/glutaraldehyde, is applied to a cavity floor in a prepared tooth as a biodegradable cavity liner and/or pulpcapping agent Upon curing, it forms a protein hydro gel forming a flexible mechanical seal thereby allowing sealing of the pulp or the RDT. Also, by containing a small stoichiometric excess of glutaraldehyde, it is strongly adhesive to collagen, thereby affording a good seal and making its margins impermeable to bacteria. Moreover, the curing process is very fast, on the order of only a few seconds, such as from 5 seconds to 60 seconds, or such as from 10 second to 45 seconds or from 15 seconds to 30 seconds, thereby minimizing time required for the application of the liner. The twopart composition, prior to mixing, includes a protein solution and an aldehyde solution. Optionally, the protein and/or the aldehyde solution can contain biocompatible particles to form a stable suspension. Examples of such particles are nanosized hydroxyapatite, mineral trioxide aggregate (MTA) and nanosilica. These particles can also be therapeutic agents in solid form such as antibiotics that can slowly dissolve resulting in a therapeutic effect. The solutions or suspensions can be dispensed by a controlled delivery system, such as one that includes a reusable delivery device, applicator tips, and applicator tip extenders. Once dispensed, the solutions are mixed in a predefined ratio in the applicator tip where cross-linking begins. By way of example, the delivery system can be similar to the ones used for 2 part cements or dental impression materials such as Mixpac ™ (Sulzer Co. Grabs Switzerland). The aldehyde molecules covalently bond (cross-link) the protein molecules to each other and, upon application, to the tissue proteins in the dentin and/or pulp, creating a flexible mechanical seal. The protein/aldehyde composition begins to polymerize within seconds, for example from 5 seconds to 60 seconds, such as from 10 second to 45 seconds or from 15 seconds to 30 seconds, and reaches its bonding strength within about 2 minutes, and generally in less than 1 minute. Good adhesion is obtained to wet surfaces, thus providing usefulness for the oral environment.

After placement and cure, the biodegradable liner composition begins to slowly biodegrade at a rate dependent on the environment. Factors affecting the rate of degradation include the rate of fluid turnover and the availability of proteolytic enzymes. As the liner degrades, it acts as a scaffold for new cell growth in the remaining space. If the liner contains particles, these can assist in reinforcing the structure of the scaffold. As a scaffold, the liner is effective for the transport of nutrients, oxygen and waste. A biodegradation time window of 2-6 months may be adequate for the regrowth of the pulp in this space.

In one embodiment, the biodegradable liner is used when the remaining dental thickness (RDT) is less than 0.5 mm. In another embodiment, the liner is particularly useful when the RDT is less than 0.2 mm. The liner is especially useful when the RDT is less than 0.2 mm and acid etching is used in the subsequent restorative procedure. The liner will protect the underlying pulp from the acid etchant thereby reducing or preventing inflammation of the dental pulp. Further, the liner is especially useful when the RDT is less than 0.2 mm and a dentin adhesive is used in the restorative procedure. The liner will protect the underlying pulp from the acid monomers and solvents, such as ethanol, that may be present in the dentin adhesive, thereby reducing or preventing inflammation of; the dental pulp.

The resorbing curable material can be used for other dental treatments or procedures in addition to its use as a cavity liner. In one aspect disclosed herein, the resorbing curable material can be used as a wound dressing after oral surgery, such as after the removal of wisdom teeth to prevent dry socket, hi another aspect disclosed herein the resorbing curable material can be used as a the resorbing curable material can be used as a biodegradable adhesive for stabilization of tooth fractures. In yet another aspect disclosed herein the resorbing curable material can be used to seal iatrogenic punctures during root canal treatment. In still other aspects disclosed herein the resorbing curable material can be used as a cover for apthlus ulcers (cancer sores) during their usual course of healing, which is typically 7-10 days, as a protective barrier over the surgical site-of dental implants and the surrounding tissue, and for protection and stabilization of bone regeneration therapy agents.

The resorbing curable compositions of the present disclosure, such as cavity liners, are based on cross-linked proteinaceous material of human or animal origin. To achieve bonding/sealing of the cavity floor or other intraoral surface, the two parts of the two-part system (the parts being referred to herein as Part A and B, respectively) are combined and the mixture is allowed to react on the surface or surfaces to be bonded/sealed and a small stoichiometric excess of glutaraldehyde is used.

Part A is an aqueous solution of a proteinaceous material of; human or animal origin, for example a solution containing about 30-55% by weight of purified or mixed protein material. Paii A can also be a suspension if particles are also included. The balance is water, dilute buffer, and/or saline solution, optionally containing non-essential materials that do not significantly interfere with the adhesive forming reaction. Examples of such non-essential materials are particles of; calcium phosphates, such as nanosized hydroxyapatite, neutral salts, carbohydrates, fibers and miscellaneous biological materials, wetting agents, antibiotics, preservatives, dyes, thickening agents, and the like. Growth factors and other soluble factors that may be active toward enhancing pulp regeneration may also be included. Albumins including ovalbumins are exemplary proteins, for example serum albumins of human or animal origin. Bovine serum albumin (BSA) is exemplary. The proteinaceous material may be a purified protein or a mixture in which the proteins, such as serum albumins, are the predominant ingredients.

Part B is a solution of a di- or polyaldehyde in water or other suitable medium. Particles such as nanosized hydroxyapatite can also be included in which case Part B is a suspension. A wide range of these substances exist, and their usefulness is restricted largely by availability and by their solubility in water. For example, aqueous glyoxal (ethanedial) is suitable, as is aqueous glutaraldehyde (pentanedial). Water soluble mixtures of di- and polyaldehydes prepared by oxidative cleavage of appropriate carbohydrates with periodate, ozone, or the like are also suitable. Glutaraldehyde is an exemplary dialdehyde ingredient for Part B, for example, glutaraldehyde in 5-15% solution. Other water-soluble di- and/or polyaldehydes may be readily recognized by persons skilled in the art for suitable use herein.

In an exemplary embodiment, the cavity liner composition is the product of cross-linking on the cavity floor a mixture initially containing 27-53 wt.% (of the mixture) of a water-soluble proteinaceous material, a di- or polyaldehyde present in a weight ratio of one part by weight to every 20-60 parts by weight of protein in the mixture, and the balance water plus optional non-essentials. By way of further example, the mixture may be formed by mixing a solution of 40-47 wt.% BSA in water and optional non-essentials with a solution of 5-15 wt.% glutaraldehyde in water and optional non-essentials in a ratio of 4:1 by volume. The final cross-linked cavity liner is a water insoluble, rubbery or leathery proteinaceous solid substantially free of aldehydes. In other words, the aldehyde is consumed by the cross-linking reaction.

However, a stoichiometric excess of aldehyde may be used, and the unreacted aldehyde may react with the dentinal surfaces to enhance adhesion or may be neutralized after polymerization, such as by exposure to a dilute solution of proteins, peptides or amino acids, for example at a 5% concentration.

When generating the cavity liner compositions or other resorbing curable compositions, the Parts A and B can be pre-mixed and applied to the cavity floor or other intraoral surface immediately following mixing by delivery means such as that described above. Alternately, Parts A and B can be applied simultaneously, as for example from a dual nozzle device and mixed on the surface(s) to be bonded. Sequential application of Parts A and B to the surfaces is also satisfactory, such as with the sequence B, then A, then B again.

In accordance with the present disclosure, a solution of a proteinaceous material and an aldehyde may be used for the manufacture of a medicament for treating an intraoral surface. The proteinaceous material and aldehyde solution is applied to the intraoral surface, and a polymerization reaction then occurs to thereby form a biodegradable material comprising the proteinaceous material in cross-linked form. In one aspect disclosed herein the intraoral surface is a fractured tooth and the biodegradable material is an adhesive formed to stabilize the fracture. In anoth aspect disclosed herein the intraoral surface is a punctured root structure and the biodegradable material is frmed to seal the puncture. In yet another aspect disclosed herein the intraoral surface is a surgical wound and the biodegradable material is formed as a wound dressing. In still another aspect disclosed herein the intraoral surface includes apthlus ulcers and the biodegradable material is formed to protect the ulcers during healing thereof. In yet anothe aspect disclosed herein the intraoral surface is a surgical site of a dental implant and the biodegradable material is formed to protect the site during healing thereof. In an embodiment, the intraoral surface is a cavity floor within a tooth and the biodegradable material is formed as a liner to protect underlying dentin and/or dental pulp.

In accordance with an exemplary embodiment, a solution of a proteinaceous material and an aldehyde is used for the manufacture of a medicament for protecting and regenerating the dental pulp in a dental restorative procedure for a tooth. The proteinaceous material and aldehyde solution is applied to a prepared cavity floor within the tooth where the RDT is less than about 0.5mm. A polymerization reaction then occurs to thereby form a biodegradable cavity liner comprising the proteinaceous material in cross-linked form to protect and regenerate dental pulp. In one further exemplary embodiment, the RDT is less than about 0.2 mm. In another further exemplary embodiment, the remaining dentin thickness between the cavity floor and the dental pulp is zero such that the cavity floor includes exposed pulp. In yet another further exemplary embodiment, the tooth area is etched with an etchant after the solution is applied, whereby the biodegradable cavity liner is formed to prevent the etchant from contacting the dental pulp. A restorative composition is then applied to the tooth area after etching to restore the tooth. In still another further exemplary embodiment, a dentin adhesive is applied to the tooth area after the solution is applied, whereby the biodegradable cavity liner is formed to prevent the dentin adhesive from infiltrating into or directly contacting the dental pulp.

In any of the above embodiments, the proteinaceous material may be bovine serum albumin or human serum albumin, and the aldehyde may be a di-aldehyde or a polyaldehyde. In exemplary embodiment, the aldehyde is glutaraldehyde and the proteinaceous material is bovine serum albumin. In another or further exemplary embodiment, prior to the solution being applied, the proteinaceous material and the aldehyde are pre-mixed to form a solution of 27-53 wt.% of the proteinaceous material and 1 part by weight of the aldehyde for every 20-60 parts by weight of the proteinaceous material, with the balance being substantially water.

It will be appreciated that various of the above-disclosed compositions and other features and functions, or alternatives thereof, may be desirably combined into many other different systems or applications. Also, various presently unforeseen or unanticipated alternatives, modifications, variations or improvements therein may be subsequently made by those skilled in the art, and are also intended to be encompassed by the following claims.

## Claims

1. A solution comprising a proteinaceous material and an aldehyde for use in a method for the protection and regeneration of dental pulp in a dental restorative procedure treating an intraoral surface, the method comprising:
(i) preparing a tooth area to provide a cavity floor within the tooth, wherein a remaining dentin thickness between the cavity floor and the dental pulp is less than 0.5 mm, and
(ii) applying to the cavity floor the solution of a proteinaceous material and an aldehyde and allowing a polymerization reaction to occur to thereby form a protein hydrogel that can perform as a biodegradable cavity liner comprising the proteinaceous material in cross-linked form of gel consistency,
(iii) etching the remaining tooth area with an etchant, and
(iv) applying a restorative composition to the tooth area after etching to restore the tooth,
wherein the proteinaceous material is an albumin.

2. The solution for use according to claim 1, wherein the proteinaceous material is bovine serum albumin, or human serum albumin.

3. The solution for use according to claim 1, wherein the aldehyde is a dialdehyde, or a polyaldehyde.

4. The solution for use according to claim 1, wherein the aldehyde is aqueous glyoxal or aqueous glutaraldehyde.

5. The solution for use according to claim 1, wherein the proteinaceous material is bovine serum albumin and the aldehyde is glutaraldehyde.

6. The solution for use according to claim 1, wherein the solution polymerizes in a time of from 5 seconds to 60 seconds.

7. The solution for use according to claim 6, wherein polymerization is complete within 2 minutes.

8. The solution for use according to claim 1, where the solution further comprises at least one of a calcium phosphate, a neutral salt, a carbohydrate, fiber, a wetting agent, an antibiotic, a preservative, a dye, a thickening agent, or a growth factor.

## Patentansprüche

1. Lösung, umfassend ein proteinhaltiges Material und einen Aldehyd zum Gebrauch bei einem Verfahren zum Schutz und zur Regeneration von Zahnpulpa bei einer Dentalrestaurationsprozedur, die eine intraorale Fläche behandelt, wobei das Verfahren umfasst:
(i) Präparieren eines Zahnbereichs, um einen Kavitätsboden innerhalb des Zahns vorzusehen, wobei eine verbleibende Dentindicke zwischen dem Kavitätsboden und der Zahnpulpa weniger als 0,5 mm beträgt, und
(ii) Aufbringen der Lösung aus einem proteinhaltigen Material und einem Aldehyd auf den Kavitätsboden und Zulassen einer Polymerisationsreaktion, um dadurch ein Proteinhydrogel zu bilden, das als biologisch abbaubare Kavitätsauskleidung, die das proteinhaltige Material in vernetzter Form einer Gelkonsistenz umfasst, fungieren kann,
(iii) Ätzen des verbleibenden Zahnbereichs mit einem Ätzmittel und
(iv) Auftragen einer Restaurationszusammensetzung auf den Zahnbereich nach dem Ätzen, um den Zahn zu restaurieren,
wobei das proteinhaltige Material ein Albumin ist.

2. Lösung zum Gebrauch nach Anspruch 1, wobei das proteinhaltige Material Rinderserumalbumin oder Humanserumalbumin ist.

3. Lösung zum Gebrauch nach Anspruch 1, wobei der Aldehyd ein Dialdehyd oder ein Polyaldehyd ist.

4. Lösung zum Gebrauch nach Anspruch 1, wobei der Aldehyd ein wässriges Glyoxal oder ein wässriger Glutaraldehyd ist.

5. Lösung zum Gebrauch nach Anspruch 1, wobei das proteinhaltige Material Rinderserumalbumin ist und der Aldehyd Glutaraldehyd ist.

6. Lösung zum Gebrauch nach Anspruch 1, wobei die Lösung in einer Zeit von 5 Sekunden bis 60 Sekunden polymerisiert.

7. Lösung zum Gebrauch nach Anspruch 6, wobei die Polymerisation innerhalb von 2 Minuten abgeschlossen ist.

8. Lösung zum Gebrauch nach Anspruch 1, wobei die Lösung ferner mindestens eines von einem Calciumphosphat, einem Neutralsalz, einem Kohlenhydrat, einer Faser, einem Benetzungsmittel, einem Antibiotikum, einem Konservierungsmittel, einem Farbstoff, einem Verdickungsmittel oder einem Wachstumsfaktor umfasst.

## Revendications

1. Solution comprenant un matériau protéique et un aldéhyde à utiliser dans un procédé pour la protection et la régénération de la pulpe dentaire dans une procédure de restauration dentaire de traitement d'une surface intraorale, le procédé comprenant :
(i) la préparation d'une zone dentaire pour fournir un plancher de cavité à l'intérieur de la dent, dans laquelle une épaisseur de dentine restante entre le plancher de la cavité et la pulpe dentaire est inférieure à 0,5 mm, et
(ii) l'application sur le plancher de la cavité de la solution d'un matériau protéique et d'un aldéhyde et le fait de permettre à une réaction de polymérisation de se produire pour ainsi former un hydrogel protéique qui peut fonctionner comme une coiffe de cavité biodégradable comprenant le matériau protéique sous forme réticulée de consistance de gel,
(iii) le mordançage de la zone restante de la dent avec un agent de mordançage, et
(iv) l'application d'une composition réparatrice sur la zone dentaire après le mordançage pour restaurer la dent,
dans lequel le matériau protéique est une albumine.

2. Solution à utiliser selon la revendication 1, dans laquelle le matériau protéique est de l'albumine sérique bovine ou de l'albumine sérique humaine.

3. Solution à utiliser selon la revendication 1, dans laquelle l'aldéhyde est un dialdéhyde, ou un polyaldéhyde.

4. Solution à utiliser selon la revendication 1, dans laquelle l'aldéhyde est du glyoxal aqueux ou du glutaraldéhyde aqueux.

5. Solution à utiliser selon la revendication 1, dans laquelle le matériau protéique est de l'albumine sérique bovine et l'aldéhyde est le glutaraldéhyde.

6. Solution à utiliser selon la revendication 1, dans laquelle la solution polymérise en un temps de 5 secondes à 60 secondes.

7. Solution à utiliser selon la revendication 6, dans laquelle la polymérisation est terminée dans les 2 minutes.

8. Solution à utiliser selon la revendication 1, où la solution comprend en outre au moins un parmi un phosphate de calcium, un sel neutre, un glucide, une fibre, un agent mouillant, un antibiotique, un conservateur, un colorant, un agent épaississant ou un facteur de croissance.
